# EUROPEAN PATENT SPECIFICATION

(11) **EP 1 789 079 B1**
(45) Date of publication and mention of the grant of the patent: **07.11.2012**
(21) Application number: 05778990.1
(22) Date of filing: 09.09.2005
(51) Int. Cl.: A61K 31/352, A61K 31/4433, A61P 25/28

(54) **Insulin-regulated aminopeptidase (IRAP) inhibitors and uses thereof**
Inhibitoren der insulinregulierten Aminopeptidase (IRAP) und ihre Verwendungen
Inhibiteurs de l'aminopeptidase régulée par l'insuline et leurs utilisations

(30) Priority: 09.09.2004 AU 2004905182
(43) Date of publication of application: 30.05.2007
(73) Proprietor: HOWARD FLOREY INSTITUTE OF EXPERIMENTAL PHYSIOLOGY AND MEDICINE, Parkville VIC 3052 (AU); ST. VINCENT'S INSTITUTE OF MEDICAL RESEARCH, Fitzroy, VIC 3065 (AU)
(72) Inventor: CHAI, Siew Yeen, Burwood East, Victoria 3151 (AU); PARKER, Michael, William, Newport, Victoria 3065 (AU); ALBISTON, Anthony, Lloyd, Brunswick East, Victoria 3057 (AU); MORTON, Craig, J., Glen Waverley, Victoria 3150 (AU); NG, Hooi Ling, Docklands, Victoria 3008 (AU); YE, Siying, North Melbourne, Victoria 3051 (AU); MENDELSOHN, Frederick, A. O., Carlton, Victoria 3053 (AU)
(74) Representative: Lamb, Martin John Carstairs
(86) International application number: PCT/AU2005/001380
(87) International publication number: WO 2006/026832

(56) References cited:
- EP-A2- 0 599 514
- WO-A1-02/092594
- WO-A1-03/011304
- US-A- 5 281 619

## Description

### FIELD OF THE INVENTION

The present invention relates to inhibitors of insulin-regulated aminopeptidase (IRAP) and methods for inhibiting same, as well as compositions comprising said inhibitors. In particular, the inhibitors of the present invention may be useful in therapeutic applications including enhancing memory and learning functions.

### DESCRIPTION OF THE PRIOR ART

Insulin-regulated aminopeptidase (IRAP) is a 165 kDa glycoprotein that is widely distributed in many tissues including fat, muscles, kidney, adrenal, lung and heart (Keller *et al.,* 1995; *Rogi et al.,* 1996; *Zhang et al.,* 1999). In the brain, it occurs as a smaller 140 kDa protein, probably due to differential glycosylation (Keller *et al.,* 1995; Zhang *et al.,* 1999). It is a type II integral membrane protein belonging to the M1 family of zinc-dependent metallopeptidases and possesses a large C-terminal extracellular tail which contains the catalytic site, a single transmembrane domain and a smaller N-terminal intracellular domain (Keller *et al.,* 1995; Rogi *et al.,* 1996). Initially cloned from a rat epididymal fat pad cDNA library as a marker protein (vp165) for a specialised vesicle containing the insulin-responsive glucose transporter GLUT4 (Keller *et al.,* 1995), the same protein was cloned concurrently from human placental cDNA library as oxytocinase (Rogi *et al.,* 1996), an enzyme which was thought to have an important role in degrading oxytocin. The AT₄ receptor has also recently been identified as the transmembrane enzyme insulin regulated aminopeptidase (IRAP) via mass spectral analysis of tryptic peptides generated from AT₄ receptor protein purified from bovine adrenal membranes *(Albiston et al.,* 2001). Analysis of the biochemical and pharmacological properties of IRAP confirm that it is, in fact, the AT₄ receptor (Albiston *et al.,* 2001). Although isolated by three independent groups from different tissue sources and thought to subserve distinct physiological roles, properties and characteristics of this protein remain consistent.

The AT₄ ligands, angiotensin IV (Ang IV), its analogues Nle-Ang IV and Norleucinal Ang IV, and the structurally distinct peptide LVV-hemorphin 7 (LVV-H7), all bind with high affinity and relative specificity to a pharmacologically distinct binding site, termed the AT₄ receptor. All the AT₄ ligands, Ang IV, Nle-Ang IV, and LVV-H7, were demonstrated in *vitro* to be inhibitors of the aminopeptidase activity of IRAP as assessed by cleavage of the synthetic substrate Leu-β-naphthylamide (Albiston *et al.,* 2001; *Lew et al.,* 2003). Both Ang IV and LVV-H7 display competitive kinetics indicating that AT₄ ligands mediate their effects by binding to the catalytic site of IRAP. Using the same system it has also been demonstrated that although the peptides Ang IV and LVV-H7 bind to the catalytic site they are not cleaved by IRAP (Lew *et al.,* 2003).

Central administration of the peptide AT₄ ligands, Ang IV, its more stable analogues, or LVV-H7, in normal animals has been shown to lead to improved performance of memory tasks in both passive avoidance and spatial learning paradigms. The initial effects were observed in the passive avoidance paradigm in rats where an intracerebro-ventricular dose (1 nmol) of Ang IV increased the latency in re-entering the dark chamber after an aversive stimulus (Braszko *et al.,* 1988; *Wright et al.,* 1993; *Wright et al.,* 1996). Chronic infusion (6 days) of an Ang IV analogue into the lateral ventricle of rats at a dose of between 0.1 and 0.5 nmol/h enhanced performance in the swim maze, a spatial memory paradigm. In the Barnes maze, another spatial learning task, treatment of rats with 100 pmoles or 1 nmol of the peptide AT₄ ligands, Nle¹-Ang IV or LVV-H7, decreased the time taken to achieve learner criteria in this paradigm (Lee *et al.,* 2004). Control animals treated with artificial cerebrospinal fluid took 7 days to achieve learner criteria, whereas animals treated with Nle¹-Ang IV or LVV-H7, at a concentration of either 100 pmoles or 1 nmole, achieved learner criteria in 3-4 days (Lee *et al.,* 2004). This observation strongly indicates that the two peptides tested not only improved memory, but also enhanced spatial learning.

Not only did peptide AT₄ ligands enhance memory and learning in normal animals, the peptides reversed memory deficits induced (1) chemically by a muscarinic antagonist or (2) mechanically by knife-cut lesion of the perforant pathway. A more stable analogue of Ang IV, Nle-Ang IV, given acutely into the lateral ventricles, reversed the memory deficits induced by the muscarinic receptor antagonist, scopolamine, in a spatial learning paradigm (Pederson *et al.,* 1998; Pederson *et al.,* 2001). In the swim maze paradigm, memory deficits induced by bilateral perforant pathway lesion can be reversed by an acute dose (1 nmol) of another Ang IV analogue, Norleucinal Ang IV (Wright *et al.,* 1999). The other AT₄ ligand, LVV-H7, given acutely prior to the conditioning trial in the passive avoidance paradigm, has also been found to reverse the memory deficit induced by scopolamine (Albiston *et al.,* 2004).

Unlike the other members of the M1 family of aminopeptidases, the N-terminal tail of IRAP is much longer (112 amino acids) and contains two dileucine motifs (residues 53-54 and 76-77) which are preceded by acidic clusters (Keller *et al.,* 1995). These characteristic sorting motifs also occur in the carboxy terminal domain of GLUT4 (Bryant *et al.,* 2002) and suggest that these two proteins undergo similar intracellular sorting processes. Microinjection of the N-terminal tail of IRAP (1-109) or a shorter fragment of the intracellular domain, IRAP (55-82), into 3T3-L1 adipocytes results in translocation of GLUT4 vesicles to the plasma membrane (Waters *et al.,* 1997) lending support for a role for the intracellular domain of IRAP in the trafficking and/or retention of GLUT4 vesicles.

As mentioned above, IRAP is not only known as a marker protein (vp165) for GLUT4 vesicles (Keller *et al.,* 1995). The same protein was cloned concurrently from human placental cDNA library as oxytocinase (Rogi *et al.,* 1996), an enzyme which was thought to have an important role in degrading oxytocin. Oxytocin and the oxytocin receptor have two important roles in labour. Evidence in all mammalian species suggests that oxytocin plays a role in the expulsive phase and is important for the precise timing of the onset of labour (Imamura *et al.,* 2000). The initiation of labour may be mediated in women and rhesus monkeys by paracrine rather than endocrine mechanisms. Studies in knockout mice also confirm Important Interrelationships between oxytocin and prostaglandins. Oxytocin stimulates prostaglandin release in many species, mainly in the decidua/uterine epithelium. The effects of oxytocin are mediated by tissue-specific oxytocin receptor expression, which leads directly to contraction in the myometrium and prostaglandin formation in the decidua. There is a dramatic increase in oxytocin receptor expression in these tissues in late pregnancy and pharmacological inhibition delays delivery, which suggests that the oxytocin receptor is essential for normal labour. Recent studies in mice with a null mutation of the oxytocin gene suggest an important role for oxytocin in milk ejection.

IRAP therefore provides a target for the development of agents which may enhance or improve memory and learning, treat disorders of glucose homeostasis, including hyperglycaemia and diabetes and/or induce labour or lactation in individuals. Accordingly, inhibitors of IRAP, which may disrupt or interfere with IRAP functional activity may have useful therapeutic and/or prophylactic applications in the treatment of conditions and disorders where excessive or undesirable IRAP activity plays a role.

Other publications include EP0599514 ("Pyranoquinoline derivatives as inhibitors of cell proliferation") and US5281619 ("Therapy for diabetic complications").

### SUMMARY OF THE INVENTION

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

It has now been found that certain benzo-fused compounds have IRAP inhibitory activity, and may therefore be useful in therapeutic applications in which IRAP inhibition is desirable.

According to the present invention, there is provided compounds for use as defined in the appended independent claim 1. Further preferable features are defined in the appended dependent claims.

Also disclosed is a method for inhibiting IRAP activity which comprises contacting IRAP with an inhibitory amount of a compound of formula (1), or a pharmaceutically acceptable salt or prodrug thereof. inhibition of IRAP activity can be performed *in vitro* or *in vivo,* preferably *in vivo* in a subject. The disclosure therefore also provides a method for inhibiting IRAP activity in a subject in need thereof, which comprises administering to said subject an inhibitory effective amount of a compound of formula (I), or a pharmaceutically acceptable salt or prodrug thereof.

Compounds described herein may be useful in treating a disease or condition in which excessive or undesirable IRAP activity plays a role. Thus, also disclosed is a method for treating a disease or condition in which IRAP activity is implicated, in a subject in need thereof, comprising the step of administering to said subject an effective amount of a compound of formula (I), or a pharmaceutically acceptable salt or prodrug thereof. The disclosure also provides for the use of a compound of formula (I), or a pharmaceutically acceptable salt or prodrug thereof in the manufacture of a medicament for treating a disease or condition in which IRAP activity is implicated.

As described above, IRAP inhibitors have been shown to improve memory and enhance spatial learning as well as reversing memory deficits.

Accordingly, also disclosed is a method for enhancing memory and/or learning in a subject, comprising the step of administering to said subject a compound of formula (I) or a pharmaceutically acceptable salt or prodrug thereof. The disclosure thus also provides for the use of a compound of formula (I), or a pharmaceutically acceptable salt or prodrug thereof in the manufacture of a medicament for enhancing memory and/or learning in a subject.

Since IRAP is involved in the trafficking of GLUT4 vesicles, the binding of AT₄ ligands to IRAP may alter this trafficking pathway thereby modulating glucose uptake. Indeed, Ang IV (0.1 and 1 uM) has been shown to enhance basal and insulin (0.7nM) stimulated glucose uptake into 3T3-L1 cells. In subjects with type II or non-insulin dependent diabetes (NIDDM), fat and muscles cells respond defectively to insulin and are unable to contribute to the maintenance of glucose homeostasis. Agents which can effectively mobilise GLUT4 vesicles to the plasma membrane or maintain GLUT4 at the plasma membrane, such as IRAP inhibitors, may be useful in the management of glucose homeostasis and/or type II diabetes including prevention of the symptoms of type II diabetes such as hyperglycaemia and other unwanted clinical sequalae.

Therefore, also disclosed is a method for treating disorders of glucose homeostasis and/or type II diabetes in a subject in need thereof, comprising the step of administering to said subject a compound of formula (I) or a pharmaceutically acceptable salt or prodrug thereof. The disclosure thus provides for the use of a compound of formula (I) or a pharmaceutically acceptable salt or prodrug thereof in the manufacture of a medicament for treating disorders of glucose homeostasis and/or type II diabetes.

Agents which may be able to extend the half-life of circulating or local oxytocin levels, such as IRAP inhibitors, may be useful in inducing labour or assisting milk ejection in lactating mothers.

Accordingly, also disclosed is a method for inducing labour or lactation in a subject in need thereof, comprising the step of administering to said subject a compound of formula (I) or a pharmaceutically acceptable salt or prodrug thereof, as well as the use of a compound of formula (I) or a pharmaceutically acceptable salt or prodrug thereof in the manufacture of a medicament for inducing labour or lactation in a subject.

Further aspects of the disclosure correspond to those above using Compound II as defined herein or a pharmaceutically acceptable salt or prodrug thereof.

### BRIEF DESCRIPTION OF THE FIGURES

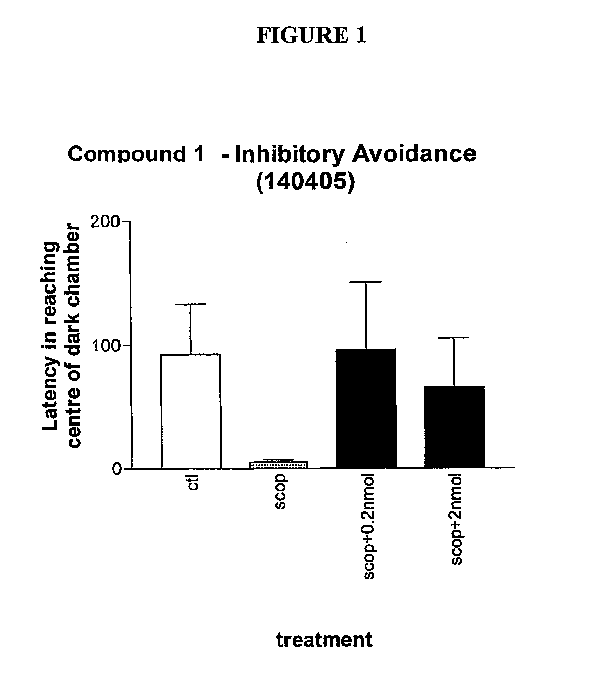

Figure 1 graphically depicts the effect of Compound 1 on scopolamine-induced memory deficit.

### DESCRIPTION OF THE INVENTION

The present invention provides compounds for use as IRAP inhibitors. Compounds disclosed herein include those of formula (I): 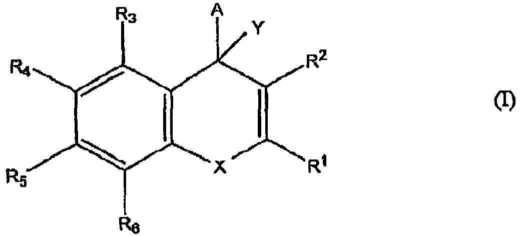 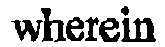
- A: is aryl heteroaryl carbocyclyl or hetezocyclyl, each of which may be optionally substituted;
- R¹: is NR⁷R⁸, NHCOR₈, N(COR₈)₂, N(COR₇)(COR₈), N=CHOR₈ or N=CHR₈ wherein R⁷ and R⁸ are independently selected from H, optionally substituted alkyl, optionally substituted aryl, or R⁷ and R⁸, together with the nitrogen atom to which they are attached form a 3-8-membered ring which may be optionally substituted;
- R²: is CN, CO₂R⁹, C(O)O(O)R⁹, C(O)R⁹ or C(O)NR⁹R¹⁰ wherein R⁹ and R¹⁰ are independently selected from alkyl, alkenyl, alkynyl, aryl, heteroaryl, carbocyclyl, heterocyclyl, each of which may be optionally substituted, and hydrogen; or R⁹ and R¹⁰, together with the nitrogen atom to which they are attached, form a 3-8-membered ring which may be optionally substituted;
- R³-R⁶: are independently selected from hydrogen, halo, nitro, cyano alkyl, alkenyl, alkynyl, aryl, heteroaryl, heterocyclyl, carbocyclyl, hydroxy, alkoxy, alkenyloxy, alkynyloxy, alkynyloxy, aryloxy, heteroaryloxy, heterocyclyloxy, amino, acyl, acyloxy, carboxy, carboxyester, methylenedioxy, amido, thio, alkylthio, alkenylthio, alkynylthio, arylthio, heteroarylthio, heterocyclylthio, carbocyclylthio, acylthio and azido, each of which may be optionally substituted where appropriate, or any two adjacent R³-R⁶, together with the atoms to which they are attached, form a 3-8-membered ring which may be optionally substituted; and
- Y: is hydrogen or C₁₋₁₀alkyl,
or a pharmaceutically acceptable salt or prodrug thereof.

As used herein, the term "alkyl", used either alone or in compound words denotes straight chain, branched or cyclic alkyl, preferably C₁₋₂₀ alkyl, eg. C₁₋₁₀ or C₁₋₆. Examples of straight chain and branched alkyl include methyl, ethyl, *n*-propyl*,* isopropyl, *n*-butyl, *sec-*butyl, t-butyl, *n*-pentyl*,* 1,2-dimethylpropyl, 1,1-dimethyl-propyl, hexyl, 4-methylpentyl, 1-methylpentyl, 2-methylpentyl, 3-methylpentyl, 1,1-dimethylbutyl, 2,2-dimethylbutyl, 3,3-dimethylbutyl, 1,2-dimethylbutyl, 1,3-dimethylbutyl, 1,2,2,-trimethylpropyl, 1,1,2-trimethylpropyl, heptyl, 5-methylhexyl, 1-methylhexyl, 2,2-dimethylpentyl, 3,3-dimethylpentyl, 4,4-dimethylpentyl, 1,2-dimethylpentyl, 1,3-dimethylpentyl, 1,4-dimethyl-pentyl, 1,2,3-trimethylbutyl, 1,1,2-trimethylbutyl, 1,1,3-trimethylbutyl, octyl, 6-methylheptyl, 1-methylheptyl, 1,1,3,3-tetramethylbutyl, nonyl, 1-, 2-, 3-, 4-, 5-, 6- or 7-methyl-octyl, 1-, 2-, 3-, 4- or 5-ethylheptyl, 1-, 2- or 3-propylhexyl, decyl, 1-, 2-, 3-, 4-, 5-, 6-, 7- and 8-methylnonyl, 1-, 2-, 3-, 4-, 5- or 6-ethyloctyl, 1-, 2-, 3- or 4-propylheptyl, undecyl, 1-, 2-, 3-, 4-, 5-, 6-, 7-, 8- or 9-methyldecyl, 1-, 2-, 3-, 4-, 5-, 6- or 7-ethylnonyl, 1-, 2-, 3-, 4- or 5-propylocytl, 1-, 2- or 3-butylheptyl, 1-pentylhexyl, dodecyl, 1-, 2-, 3-, 4-, 5-, 6-, 7-, 8-, 9- or 10-methylundecyl, 1-,2-,3-,4-, 5-, 6-, 7- or 8-ethyldecyl, 1-, 2-, 3-, 4-, 5- or 6-propylnonyl, 1-,2-,3- or 4-butyloctyl, 1-2-pentylheptyl and the like. Examples of cyclic alkyl include mono- or polycyclic alkyl groups such as cyclopropyl, cyclobutyl, cyclopentyl, cyclohexyl, cycloheptyl, cyclooctyl, cyclononyl, cyclodecyl and the like. Where an alkyl group is referred to generally as "propyl", butyl" etc, it will be understood that this can refer to any of straight, branched and cyclic isomers where appropriate. An alkyl group may be optionally substituted by one or more optional substitutents as herein defined.

The term "alkenyl" as used herein denotes groups formed from straight chain, branched or cyclic hydrocarbon residues containing at least one carbon to carbon double bond including ethylenically mono-, di- or poly-unsaturated alkyl or cycloalkyl groups as previously defined, preferably C₂₋₂₀ alkenyl (eg. C₂₋₁₀ or C₂₋₆). Examples of alkenyl include vinyl, allyl, 1-methylvinyl, butenyl, iso-butenyl, 3-methyl-2-butenyl, 1-pentenyl, cyclopentenyl, 1-methyl-cyclopentenyl, 1-hexenyl, 3-hexenyl, cyclohexenyl, 1-heptenyl, 3-heptenyl, 1-octenyl, cyclooctenyl, 1-nonenyl, 2-nonenyl, 3-nonenyl, 1-decenyl, 3-decenyl, 1,3-butadienyl, 1-4,pentadienyl, 1,3-cyclopentadienyl, 1,3-hexadienyl, 1,4-hexadienyl, 1,3-cyclohexadienyl, 1,4-cyclohexadienyl, 1,3-cycloheptadienyl, 1,3,5-cycloheptatrienyl and 1,3,5,7-cyclooctatetraenyl. An alkenyl group may be optionally substituted by one or more optional substitutents as herein defined.

As used herein the term "alkynyl" denotes groups formed from straight chain, branched or cyclic hydrocarbon residues containing at least one carbon-carbon triple bond including ethylenically mono-, di- or poly- unsaturated alkyl or cycloalkyl groups as previously defined. Unless the number of carbon atoms is specified the term preferably refers to C₂₋₂₀ alkynyl (eg. C₂₋₁₀ or C₂₋₆). Examples include ethynyl, 1-propynyl, 2-propynyl, and butynyl isomers, and pentynyl isomers. An alkynyl group may be optionally substituted by one or more optional substituents as herein defined.

Terms written as "[group]oxy" refer to a particular group when linked by oxygen, for example, the terms "alkoxy", "alkenoxy",. "alkynoxy" and "aryloxy" and "acyloxy" respectively denote alkyl, alkenyl, alkynyl, aryl and acyl groups as hereinbefore defined when linked by oxygen. Terms written as "[group]thio" refer to a particular group when linked by sulfur, for example, the terms "alkylthio", "alkenylthio", alkynylthio" and "arylthio" respectively denote alkyl, alkenyl, alkynyl, aryl groups as hereinbefore defined when linked by sulfur.

The term "halogen" ("halo") denotes fluorine, chlorine, bromine or iodine (fluoro, chloro, bromo or iodo).

The term "aryl" denotes any of single, polynuclear, conjugated and fused residues of aromatic hydrocarbon ring systems. Examples of aryl include phenyl, biphenyl, terphenyl, quaterphenyl, naphthyl, tetrahydronaphthyl, anthracenyl, dihydroanthracenyl, benzanthracenyl, dibenzanthracenyl, phenanthrenyl, fluorenyl, pyrenyl, idenyl, azulenyl, chrysenyl. Preferred aryl include phenyl and naphthyl. An aryl group may be optionally substituted by one or more optional substituents as herein defined.

The term "carbocyclyl" includes any of non-aromatic monocyclic, polycyclic, fused or conjugated hydrocarbon residues, preferably C₃₋₂₀ (eg. C₃₋₁₀ or C₃₋₈). The rings may be saturated, eg. cycloalkyl, or may possess one or more double bonds (cycloalkenyl) and/or one or more triple bonds (cycloalkynyl). Particularly preferred carbocyclyl are 5-6-membered or 9-10 membered ring systems. Suitable examples include cyclopropyl, cyclobutyl, cyclopentyl, cyclohexyl, cycloheptyl, cyclooctyl, cyclononyl, cyclodecyl, cyclopentenyl, cyclohexenyl, cyclooctenyl, cyclopentadienyl, cyclohexadienyl, cyclooctatetraenyl, indanyl, decalinyl and indenyl.

The term "heterocyclyl" or "heterocyclic" when used alone or in compound words includes any of monocyclic, polycyclic, fused or conjugated hydrocarbon residues, preferably C₃₋₂₀ (eg. C₃₋₁₀ or C₃₋₈) wherein one or more carbon atoms are replaced by a heteroatom so as to provide a non-aromatic residue. Suitable heteroatoms include, O, N, S, P and Se, particularly O, N and S. Where two or more carbon atoms are replaced, this may be by two or more of the same heteroatom or by different heteroatoms. The heterocyclyl group may be saturated or partially unsaturated, ie. possess one or more double bonds. Particularly preferred heterocyclyl are 5-6 and 9-10 membered heterocyclyl. Suitable examples of heterocyclyl groups may include pyrrolidinyl, pyrrolinyl, piperidyl, piperazinyl, morpholinyl, indolinyl, imidazolidinyl, imidazolinyl, pyrazolidinyl, thiomorpholinyl, dioxanyl, tetrahydrofuranyl, tetrahydropyranyl, tetrahydropyrrolyl, pyranyl and dihydropyranyl. A heterocyclyl group may be optionally substituted.

The term "heteroaryl" includes any of monocyclic, polycyclic, fused or conjugated hydrocarbon residues, wherein one or more carbon atoms are replaced by a heteroatom so as to provide an aromatic residue. Preferred heteroaryl have 3-20 ring atoms, eg. 3-10. Particularly preferred heteroaryl are 5-6 and 9-10 membered ring systems. Suitable heteroatoms include, O, N, S, P and Se, particularly O, N and S. Where two or more carbon atoms are replaced, this may be by two or more of the same heteroatom or by different heteroatoms. Suitable examples of heteroaryl groups may include pyridyl, pyrrolyl, thienyl, imidazolyl, furanyl, benzothienyl, isobenzothienyl, benzofuranyl, isobenzofuranyl, indolyl, isoindolyl, pyrazolyl, pyrazinyl, pyrimidinyl, pyridazinyl, indolizinyl, quinolyl, isoquinolyl, phthalazinyl, 1,5-naphthyridinyl, quinozalinyl, quinazolinyl, quinolinyl, oxazolyl, thiazolyl, isothiazolyl, isoxazolyl, triazolyl, oxadialzolyl, oxatriazolyl, triazinyl, and furazanyl. A heteroaryl group may be optionally substituted.

The term "acyl" either alone or in compound words denotes a group containing the moiety C=O (and not being a carboxylic acid, ester or amide) Preferred acyl includes C(O)-R, wherein R is hydrogen or an alkyl, alkenyl, alkynyl, aryl, heteroaryl, carbocyclyl, or heterocyclyl residue. Examples of acyl include formyl, straight chain or branched alkanoyl (eg. C₁₋₂₀) such as, acetyl, propanoyl, butanoyl, 2-methylpropanoyl, pentanoyl, 2,2-dimethylpropanoyl, hexanoyl, heptanoyl, octanoyl, nonanoyl, decanoyl, undecanoyl, dodecanoyl, tridecanoyl, tetradecanoyl, pentadecanoyl, hexadecanoyl, heptadecanoyl, octadecanoyl, nonadecanoyl and icosanoyl; cycloalkylcarbonyl such as cyclopropylcarbonyl cyclobutylcarbonyl, cyclopentylcarbonyl and cyclohexylcarbonyl; aroyl such as benzoyl, toluoyl and naphthoyl; aralkanoyl such as phenylalkanoyl (eg. phenylacetyl, phenylpropanoyl, phenylbutanoyl, phenylisobutylyl, phenylpentanoyl and phenylhexanoyl) and naphthylalkanoyl (eg. naphthylacetyl, naphthylpropanoyl and naphthylbutanoyl]; aralkenoyl such as phenylalkenoyl (eg. phenylpropenoyl, phenylbutenoyl, phenylmethacryloyl, phenylpentenoyl and phenylhexenoyl and naphthylalkenoyl (eg. naphthylpropenoyl, naphthylbutenoyl and naphthylpentenoyl); aryloxyalkanoyl such as phenoxyacetyl and phenoxypropionyl; arylthiocarbamoyl such as phenylthiocarbamoyl; arylglyoxyloyl such as phenylglyoxyloyl and naphthylglyoxyloyl; arylsulfonyl such as phenylsulfonyl and napthylsulfonyl; heterocycliccarbonyl; heterocyclicalkanoyl such as thienylacetyl, thienylpropanoyl, thienylbutanoyl, thienylpentanoyl, thienylhexanoyl, thiazolylacetyl, thiadiazolylacetyl and tetrazolylacetyl; heterocyclicalkenoyl such as heterocyclicpropenoyl, heterocyclicbutenoyl, heterocyclicpentenoyl and heterocyclichexenoyl; and heterocyclicglyoxyloyl such as thiazolyglyoxyloyl and thienylglyoxyloyl. The R residue may be optionally substituted as described herein.

The term, "amino" is used here in its broadest sense as understood in the art and includes groups of the formula NR^{A}R^{B} wherein R^{A} and R^{B} may be any independently selected from hydrogen, alkyl, alkenyl, alkynyl, aryl, carbocyclyl, hateroary, heterocyclyl, aralkyl, and acyl. R^{A} and R^{B}, together with the nitrogen to which they are attached, may also form a monocyclic, or polycyclic ring system eg. a 3-10 membered ring, particularly, 5-6 and 9-10 membered systems. Examples of "amino" include NH₂, NHalkyl (eg. C₁₋₂₀alkyl), NHaryl (eg. NHphenyl), NHaralkyl (eg. NHbenzyl), NHacyl (eg. NHC(O)C₁₋₂₀alkyl, NHC(O)phenyl), Nalkylalkyl (wherein each alkyl, for example C₁₋₂₀, may be the same or different) and 5 or 6 membered rings, optionally containing one or more same or different heteroatoms (eg. O, N and S).

The term "amido" is used here in its broadest sense as understood in the art and includes groups having the formula C(O)NR^{A}R^{B}, wherein R^{A} and R^{B} are as defined as above. Examples of amido include C(O)NH₂, C(O)NHalkyl (eg. C₁₋₂₀alkyl), C(O)NHaryl (eg. C(O)NHphenyl), C(O)NHaralkyl (eg. C(O)NHbenzyl), C(O)NHacyl (eg. C(O)NHC(O)C₁₋₂₀alkyl, C(O)NHC(O)phenyl), C(O)Nalkylalkyl (wherein each alkyl, for example C₁₋₂₀, may be the same or different) and 5 or 6 membered rings, optionally containing one or more same or different heteroatoms (eg. O, N and S).

The term "carboxy ester" is used here in its broadest sense as understood in the art and includes groups having the formula CO₂R, wherein R may be selected from groups including alkyl, alkenyl, alkynyl, aryl, carbocyclyl, heteroaryl, heterocyclyl, aralkyl, and acyl. Examples of carboxy ester include CO₂C₁₋₂₀alkyl, CO₂aryl (eg. CO₂phenyl), CO₂arakyl (eg. CO₂ benzyl).

In this specification "optionally substituted" is taken to mean that a group may or may not be further substituted or fused (so as to form a condensed polycyclic group) with one, two, three or more of organic and inorganic groups, including those selected from: alkyl, alkenyl, alkynyl, carbocyclyl, aryl, heterocyclyl, heteroaryl, acyl, aralkyl, alkaryl, alkheterocyclyl, alkheteroaryl, alkcarbocyclyl, halo, haloalkyl, haloalkenyl, haloalkynyl, haloaryl, halocarbocyclyl, haloheterocyclyl, haloheteroaryl, haloacyl, haloaryalkyl, hydroxy, hydroxyalkyl, hydroxyalkenyl, hydroxyalkynyl, hydroxycarbocyclyl, hydroxyaryl, hydroxyheterocyclyl, hydroxyheteroaryl, hydroxyacyl, hydroxyaralkyl, alkoxyalkyl, alkoxyalkenyl, alkoxyalkynyl, alkoxycarbocyclyl, alkoxyaryl, alkoxyheterocyclyl, alkoxyheteroaryl, alkoxyacyl, alkoxyaralkyl, alkoxy, alkenyloxy, alkynyloxy, aryloxy, carbocyclyloxy, aralkyloxy, heteroaryloxy, heterocyclyloxy, acyloxy, haloalkoxy, haloalkenyloxy, haloalkynyloxy, haloaryloxy, halocarbocyclyloxy, haloaralkyloxy, haloheteroaryloxy, haloheterocyclyloxy, haloacyloxy, nitro, nitroalkyl, nitroalkenyl, nitroalkynyl, nitroaryl, nitroheterocyclyl, nitroheteroayl, nitrocarbocyclyl, nitroacyl, nitroaralkyl, amino (NH₂), alkylamino, dialkylamino, alkenylamino, alkynylamino, arylamino, diarylamino, aralkylamino, diaralkylamino, acylamino, diacylamino, heterocyclamino, heteroarylamino, carboxy, carboxyester, amido, alkylsulphonyloxy, arylsulphenyloxy, alkylsulphenyl, arylsulphenyl, methylenedioxy thio, alkylthio, alkenylthio, alkynylthio, arylthio, aralkylthio, carbocyclylthio, heterocyclylthio, heteroarylthio, acylthio, cyano, sulfate and phosphate groups. Optional substitution may also be taken to refer to where a CH₂ group in a chain or ring is replaced by a carbonyl group (C=O).

Preferred optional substitutents include alkyl, (eg. C₁₋₆alkyl such as methyl, ethyl, propyl, butyl, cyclopropyl, cyclobutyl, cyclopentyl or cyclohexyl), hydroxyalkyl (eg. hydroxymethyl, hydroxyethyl, hydroxypropyl), alkoxyalkyl (eg. methoxymethyl, methoxyethyl, methoxypropyl, ethoxymethyl, ethoxyethyl, ethoxypropyl etc) alkoxy (eg. C₁₋₆ alkoxy such as methoxy, ethoxy, propoxy, butoxy, cyclopropoxy, cyclobutoxy), halo, trifluoromethyl, trichloromethyl, tribromomethyl hydroxy, phenyl (which itself may be further substituted eg., by C₁₋₆alkyl, halo, hydroxy hydroxyC₁₋₆alkyl, C₁-₆alkoxy, haloC₁-₆alkyl, cyano, nitro OC(O)C₁-₆alkyl, and amino), benzyl (wherein benzyl itself may be further substituted eg., by C₁₋₆alkyl, halo, hydroxy hydroxyC₁₋₆alkyl, C₁₋₆alkoxy, haloC₁₋₆alkyl, cyano, nitro OC(O)C₁₋₆alkyl, and amino), phenoxy (wherein phenyl itself may be further substituted eg., by C₁₋₆alkyl, halo, hydroxy hydroxyC₁₋₆alkyl, C₁₋₆alkoxy, haloC₁₋₆alkyl, cyano, nitro OC(O)C₁₋₆alkyl, and amino), benzyloxy (wherein benzyl itself may be further substituted eg., by C₁₋₆alkyl, halo, hydroxy hydroxyC₁₋₆alkyl, C₁₋₆alkoxy, haloC₁₋₆alkyl, cyano, nitro OC(O)C₁₋₆alkyl, and amino), amino, alkylamino (eg. C₁₋₆alkyl, such as methylamino, ethylamino, propylamino etc), dialkylamino (eg. C₁₋₆alkyl, such as dimethylamino, diethylamino, dipropylamino), acylamino (eg. NHC(O)CH₃), phenylamino (wherein phenyl itself may be further substituted eg., by C₁₋₆alkyl, halo, hydroxy hydroxyC₁₋₆alkyl, C₁₋₆alkoxy, haloC₁₋₆alkyl, cyano, nitro OC(O)C₁₋₆alkyl, and amino), nitro, formyl, -C(O)-alkyl (eg. C₁₋₆ alkyl, such as acetyl), O-C(O)-alkyl (eg. C₁₋₆alkyl, such as acetyloxy), benzoyl (wherein the phenyl group itself may be further substituted eg., by C₁₋₆alkyl, halo, hydroxy hydroxyC₁₋₆alkyl, C₁₋₆alkoxy, haloC₁₋₆alkyl, cyano, nitro OC(O)C₁₋₆alkyl, and amino), replacement of CH₂ with C=O, CO₂H, CO₂alkyl (eg. C₁₋₆ alkyl such as methyl ester, ethyl ester, propyl ester, butyl ester), CO₂phenyl (wherein phenyl itself may be further substituted eg., by C₁₋₆alkyl, halo, hydroxy hydroxyC₁₋₆alkyl, C₁₋₆alkoxy, haloC₁₋₆alkyl, cyano, nitro OC(O)C₁₋₆alkyl, and amino), CONH₂, CONHphenyl (wherein phenyl itself may be further substituted eg., by C₁₋₆alkyl, halo, hydroxy hydroxyC₁₋₆alkyl, C₁₋₆alkoxy, haloC₁₋₆alkyl, cyano, nitro OC(O)C₁₋₆alkyl, and amino), CONHbenzyl (wherein benzyl itself may be further substituted eg., by C₁₋₆alkyl, halo, hydroxy hydroxyC₁₋₆alkyl, C₁₋₆alkoxy, haloC₁₋₆alkyl, cyano, nitro OC(O)C₁₋₆alkyl, and amino),CONHalkyl (eg. C₁₋₆ alkyl such as methyl ester, ethyl ester, propyl ester, butyl amide) and CONHdialkyl (eg. C₁₋₆alkyl).

Preferred A of formula (I) include optionally substituted 5-6-membered aryl, heteroaryl, carbocyclyl and heterocyclyl, eg. phenyl, pyridyl (attached at C2, C3 or C4), pyrazinyl, pyrimidinyl (attached at C2, C4 or C5), pyridazinyl (attached at C3 or C4), s-triazinyl (attached at C2, C4 or C6), as-triazinyl (attached at C3, C5 or C6), v-triazinyl (attached at C4, C5 or C6), furanyl (attached at C2 or C3), pyrrolyl (attached at C2 or C3), thienyl (attached at C2 or C3), cyclopentyl, cyclohexyl, cyclopentadienyl, cyclohexadienyl, pyranyl, piperidinyl, piperazinyl, morpholinyl, pyrolidinyl and pyrrolinyl. In preferred embodiments A is optionally substituted 5-6-membered aryl or optionally substituted heteroaryl, particularly optionally substituted phenyl or optionally substituted pyridyl.

Particularly preferred substituents for A include alkyl, haloalkyl (eg. mono-, di- or trifluoromethyl, mono-, di-or trichloromethyl and mono-, di-, tri-, tetra, penta- or hexafluoroethyl and mono-, di-, tri-, tetra, penta- or hexachloroethyl), hydroxyalkyl, aminoalkyl, thioalklyl, arylalkyl (eg. benzyl and phenylethyl), alkoxy, haloalkoxy, hydroxyalkoxyl, aminoalkoxy, alkythio, haloalkythio, hydroxyalkylthio, aminoalkylthio, thioalkythio, amino, C(O)alkyl, OC(O)alkyl, aryl (eg. phenyl), carboxy, carboxy ester (eg. CO₂alkyl, CO₂aryl), C(O)aryl, OC(O)aryl, nitro, cyano, heteroaryl (eg. pyridyl), heteroarylalkyl, heterocyclyl, heterocyclylalkyl, hydroxy, thio, methylenedioxy, halo (eg. Cl, Br) and amido. Where such a substituent contains an "alkyl" moiety, preferred alkyl are C₁₋₁₀alkyl, particularly C₁₋₆alkyl, such as methyl, ethyl, *i*-propyl, *n*-propyl, *n*-butyl, sec-butyl or *t*-butyl.

In one embodiment X is O.

In a particularly preferred embodiment, Y is hydrogen.

Preferred R⁷ and R⁸ include hydrogen, C₁₋₁₀ alkyl, and phenyl wherein C₁₋₁₀ alkyl and phenyl may be optionally substituted.

Particularly preferred substituents for R⁷ and R⁸ (including preferred R⁷ and R⁸ as above) include alkyl, haloalkyl (eg. mono-, di- or trifluoromethyl, mono-, di-or trichloromethyl and mono-, di-, tri-, tetra, penta- or hexafluoroethyl and mono-, di-, tri-, tetra, penta- or hexachloroethyl), hydroxyalkyl, aminoalkyl, thioalkyl, arylalkyl (eg. benzyl and phenylethyl) alkoxy, haloalkoxy, hydroxyalkoxyl, aminoalkoxy alkythio, haloalkythio, hydroxyalkylthio, aminoalkylthio, thioalkythio, amino, C(O)alkyl OC(O)alkyl, aryl (eg. phenyl) carboxy, carboxy ester C(O)aryl OC(O)aryl, nitro, cyano, heteroaryl (eg. pyridyl) heteroarylalkyl, heterocyclyl, heterocyclylalkyl, hydroxy, thio, methylenedioxy, halo (eg. Cl, Br) and amido or together form a 5-6-membered ring eg. (piperidyl, morpholinyl). Where such a substituent contains an "alkyl" moiety, preferred alkyl are C₁₋₁₀alkyl, particularly C₁₋₆alkyl. Particularly preferred R¹ include NH₂, NHC₁₋₆alkyl, N(C₁₋₆alkyl) (C₁₋₆alkyl) and NHC(O)C₁₋₆alkyl, most preferably NH₂.

For R², Preferred R⁹ and R¹⁰ include hydrogen, C₁₋₁₀ alkyl, aryl (eg. phenyl) heteroaryl (eg. pyridyl). One particularly preferred R² is cyano (CN). Other preferred R² are CO₂C₁₋₁₀ alkyl, (eg. CO₂Me, CO₂Et, CO₂Pr, CO₂Bu etc) and amido, eg. CONH₂.

Examples of suitable R³-R⁶ include hydrogen, chloro, bromo, C₁₋₁₀ alkyl (including cycloalkyl), C₂₋₁₀ alkenyl (including cycloalkenyl), C₂₋₁₀ alkynyl (including cycloalkynyl), C₁₋₁₀ alkoxy (eg. methoxy, ethoxy, *n*- and *i*-propoxy and *n*-, sec- and t-butoxy), phenyl, halophenyl, hydroxyphenyl, aminophenyl, alkylphenyl, hydroxy, NH₂, NHC₁₋₁₀alkyl, N C₁₋₁₀alkylC₁₋₁₀alkyl (wherein each alkyl may be the same or different), nitro, haloalkyl, including trifluromethyl, trichloromethyl, acyl (eg. C(O)C₁₋₁₀alkyl), acyloxy (eg. OC(O)C₁₋₁₀alkyl or OC(O)aryl such as OC(O)phenyl), carboxy ester (eg. CO₂C₁₋₁₀alkyl and CO₂ phenyl), CO₂H, amido (eg. CONHC₁₋₁₀alkyl), nitro, cyano, thio, alkylthio (eg. SC₁₋₁₀alkyl) and 2 of adjacent R³-R⁶ form methylenedioxy. None, one, two, three or four of R³-R⁶ may be hydrogen. In one preferred form, 2 or 3 or 4 of R³-R⁶ are hydrogen. In one particular embodiment, R³, R⁴ and R⁶ are all H.

Where any two adjacent R³-R⁶ form a 3-8-membered ring together with the atoms to which they are attached, the ring may be carbocylic (saturated or partially unsaturated), heterocyclic, aryl or heteroaryl. In some preferred embodiments, the ring formed has 5-6-members. Particularly preferred forms of this embodiment are where R⁵ and R⁶ form a ring. Examples of any two adjacent R³-R⁶ taken together include -(CH₂)ₙ- where n is 1-7, preferably 1-4, particularly 3 or 4, -O-CH₂-O-, O-(CH₂)₂-O -CH=CH₂-CH₂=CH-, -CH₂-NHCH₂₋, -(CH₂)₂-NH-CH₂-, -CH₂-NH-(CH₂)₂-, -(CH₂)₂-NH-, -NH-(CH₂)₂-, -NH-CH=CH-, -CH=CH-NH-, -O-CH=CH-, -CH=CH-O-, -S-CH=CH-, -CH=CH-S-, -N=CH-CH=N- O-CH=CH-CH₂-, and -CH₂-CH=CH-O-. Where appropriate, an N atom within such a ring may be further substituted with alkyl (eg. C₁₋₁₀), aryl (eg. phenyl), arylalkyl (eg. benzyl or phenylethyl), acyl (eg. C(O)C₁₋₁₀alkyl) hydroxyalkyl (eg. C₁₋₁₀), haloalkyl (eg. C₁₋₁₀), carbocyclylalkyl, heteroarylalkyl, heterocyclylalkyl, carbocyclyl, heteroaryl or heterocyclyl.

Other compounds which may have utility as IRAP inhibitors and/or therapeutic agents according to the invention are further described and/or exemplified in WO 02/092594.

Compounds for use in the invention may be obtained through commercial sources or prepared via methods known in the art of synthetic organic chemistry, see for example WO 02/092594.

Thus, for the preparation of compounds where X is O, an appropriately substituted phenol compound may be reacted with an appropriate aldehyde and malononitrile in the presence of a base such as piperidine or N,N-diisopropylethylamine in accordance with the generalised Scheme 1 below:

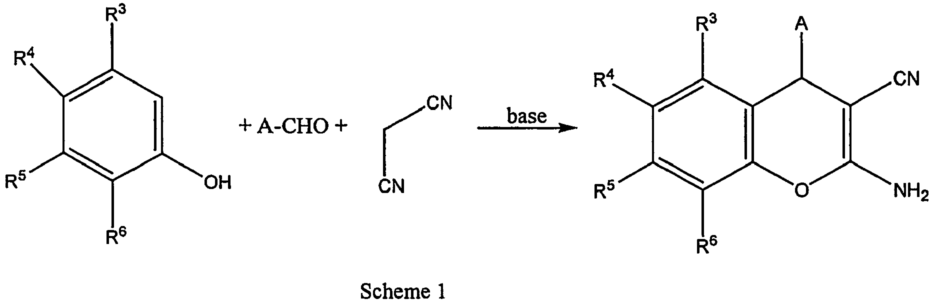

Alternatively, the aldehyde may be reacted with malononitrile in the presence of a base, and the resulting arylidene intermediate reacted with the appropriate phenol.

In an alternative methodology, the appropriately substituted *ortho*-aroyl phenol, aniline or benzenethiol can be reacted with malononitrile (or appropriate cyanoacetate, cyanoacetamide or acylacetonitrile) to access the corresponding 4*H*-chromene, 1,4-dihydroquinoline or 4*H*-thiochromene as in Scheme 2 below (where P is H or a protecting group as appropriate).

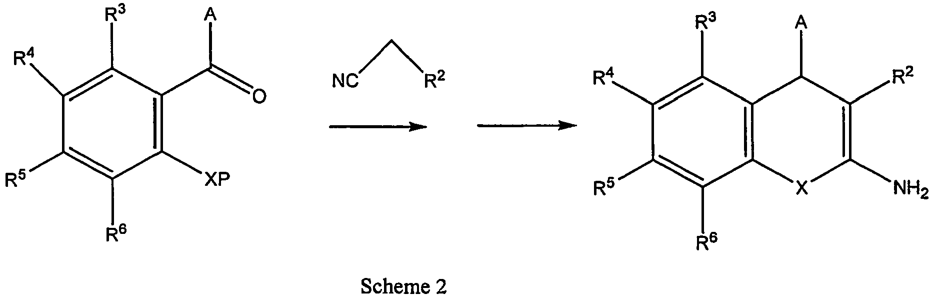

Suitable dihydroquinoline compounds can also be prepared by reduction of an appropriately substituted or protected quinoline.

Compounds of formula (I), where R¹ is other than NH₂ may be prepared by converting the amine to the desired group using chemical transformations known in the art, for example as described in Comprehensive Organic Transformation, A Guide to Functional Group Preparations, R. C. Larock, VCH, 1989 *and* Advanced Organic Chemistry, Reactions, Mechanisms and Structure, J. March, 3rd Edition, 1985 or 4th Edition, 1992 (the entire contents of which are incorporated herein by reference). Thus, treatment of the amine (NH₂) with a suitable acylating agent such as a carboxylic acid, anhydride or chloride in the presence of an appropriate base or catalyst provides access to compounds of formula (I) where R¹ is NHCOR⁸. Alkyl and aryl amines can be prepared by treatment of -NH₂ with an appropriate alkyl or aryl halide. Imines may be formed by treating the amine group (NH₂) with a suitable carbonyl containing compound such as an aldehyde or ketone in accordance with art known methods.

Similarly, compounds where R² is other than CN, ie. carboxylic acid, esters, amides, anhydrides and ketones, may be prepared by transformations known in the art, see in particular Larock, *supra,* Chapter 9, pp 963-995. Alternatively, reaction of the appropriate phenol, aldehyde and the appropriate cyanoacetate, cyanoacetamide or acylacetonitrile affords access to compounds where R² is CO₂R⁹, C(O)NR⁹R¹⁰, C(O)R⁹ and C(O)O(O)R⁹. Compounds where Y is alkyl can be prepared by treating the benzopyran with DDQ and the subsequent intermediate with CuBr·DMS and an alkyllithium compound.

It will be recognised that during the processes for the preparation of compounds contemplated by the present invention, it may be necessary or desirable to protect certain functional groups which may be reactive or sensitive to the reaction or transformation conditions undertaken (eg. OH, NH₂, CO₂H, SH, C=O). Suitable protecting groups for such functional groups are known in the art and may be used in accordance with standard practice. Such protecting groups and methods for their installation and subsequent removal at an appropriate stage are described *in* Protective Groups in Organic Chemistry, 3rd Edition, T.W.Greene and P. G. Wutz, John Wiley and Sons, 1999, the entire contents of which are incorporated herein by reference.

The level of IRAP inhibitory activity of the compounds disclosed herein can be initially determined in an *in vitro* assay, which measures the ability of the test compound to inhibit the aminopeptidase activity of IRAP, by assessing the rate or extent of cleavage or degradation of an IRAP aminopeptidase substrate such as Leu-β-naphthylamide or Leu-4-methylcoumaryl-7-amide. Comparison can then be made to a control assay, whereby the rate or extent of cleavage is determined in the absence of the compound. A comparative reduction in the rate or extent of cleavage of the substrate in the presence of the compound can be taken to be a measure of the inhibitory effect of the compound.

Disorders and conditions where undesirable or excessive IRAP aminopeptidase activity is implicated or involved may include memory or learning disorders associated with Alzheimer's disease and other forms of dementia and memory loss (be they age-related, induced through head trauma, hypoxic damage, surgery, cerebral infarcts or chemical means such as neurotoxins). It should also be appreciated that the compounds of the present invention may also be useful in enhancing or improving memory or learning in normal individuals, ie. those not suffering from cognitive pathologies such as those described above.

Memory or learning (eg spatial learning) enhancement refers to an improvement in the ability of a subject to memorize or learn information and can be determined by well established tests. A positive improvement in the "score" or result obtained in such a test compared to a score/result obtained prior to administration of the compounds is taken to be an enhancement in memory or learning as appropriate.

A number of well known and established tests exist for laboratory animals and rats and mice can be tested using these, which include the Barnes Maze paradigm (Greferath *et al.,* 2000), Barnes Circular Maze test (Lee *et al.,* 2004) or modifications thereof, the Y maze test, or passive avoidance test. These are as briefly outlined below.

### 1. Barnes maze

Normal male Sprague-Dawley rats are implanted with cannulas in the lateral ventricles and allowed to recover for at least three days. For the Barnes Circular Maze test, the maze comprises a raised rotatable white circular platform of diameter 1.2m, with 18 evenly spaced holes in the periphery (diameter 0.09m). An escape tunnel comprising a black box of internal diameter of 0.16m width, 0.29m length and 0.09m depth is positioned immediately beneath a peripheral hole. Visual cues are placed at various positions around the maze.

For each trial, the animal is placed on the maze platform under the starting chamber, which is a cylindrical chamber located in the centre of the platform, and left for 20s. Following the twenty seconds disorientation period the chamber is raised. The animals are then allowed 240s in which to find and utilise the escape tunnel. Each rat receives three consecutive trials per day over ten days. On the first day of the testing period, each rat is placed directly into the escape tunnel for a 2min familiarisation period. The rat is then replaced into its cage for approximately one minute, after which the animal is injected with either the test compound or artificial cerebrospinal fluid and then replaced into its cage for a further 5min. Three consecutive trials are then carried out, with a recovery period of 2min between each trial in its home cage. On subsequent days (days 2 to 8), the familiarisation period prior to the three trials is eliminated from the protocol.

Animals can be administered the IRAP inhibitors intracerebroventricularly (icv) via a chronic indwelling cannula. These compounds are given 5 mins before the first trial on the first day of testing.

### 2. Y maze

The Y-maze test is another behavioural paradigm which measures spatial memory performance, and exploits the natural instinct of rodents to explore novel environments.

The Y maze consists of three identical alleys (30cm in length, 10cm in width and 17cm in height) with the three arms separated by 120° angles. To minimize stress, the maze is located in a sound-attenuated room under dim illumination, with the floor of the maze covered with sawdust. After each trial, the sawdust is mixed to eliminate olfactory cues. For spatial orientation, visual cues are placed on the walls of the testing room.

The test consists of two trials, separated by a time interval known as the intertrial interval. During the acquisition trial, the animals are placed at the distal end of one arm, their heads pointing away from the centre of the maze. The animals are allowed to visit only two accessible arms of the maze for 3 minutes. At the end of the acquisition trial, animals are replaced in their cages for the intertrial interval. During the retention trial, the animals have access to all three arms for 5 minutes. The first arm entered (novel vs familiar), the number of entries and duration of time spent in the novel arm is documented.

A shorter intertrial interval (2 hours) separating the acquisition phase from the retention phase enables amnesiant effects to be detected. Control animals still remember the location of the novel arm and will preferentially spend more time (45-50% of time) in that arm. A longer intertrial interval (eg. 6 hours) results in the animals not remembering the location of the novel arm and hence spending equal amount of time in all three arms.

Animals are surgically implanted with an infusion cannula in the dorsal third ventricle. Each animal is tested twice, and the intertrial interval of greater than 6h can be adopted to test for memory-enhancing properties of the test compounds. Animals are administered the IRAP inhibitors intracerebroventricularly via a chronic indwelling cannula. These compounds are given 5 mins before the first acquisition trial. The animals are then returned to their home cages for at least 6 hours and then tested again. The time spent in the novel arm will be a measure of the memory enhancing properties of the test compound.

### 3. Passive avoidance

Passive avoidance trials can be used to test the effect of IRAP inhibitors on aversive conditioning behaviour in amnesic animals. The passive avoidance task involves aversive conditioning behaviour to measure facilitation of memory retention and retrieval. The testing can be carried out in an apparatus that consists of a light and a dark compartment separated by a guillotine door. The floor of the dark compartment contains an electrified grid. The passive avoidance task is divided into two trials separated by a 24-48h inter-trial interval. During the first trial, known as the acquisition trial, the animal is placed in the lit compartment and the guillotine door is closed once the animal enters the dark compartment. Inside the dark chamber, the animal receives a low-level electric shock (0.5mA for 2s) via the grid floor. The animal is then be returned to its home cage for 24h or 48h before being tested. The latency periods to re-enter the dark compartment are taken as a measure of the ability of the animals to remember the aversive stimuli.

Animals are to be surgically implanted with an infusion cannula in the dorsal third ventricle. Each animal is tested twice, and the intertrial interval of 24h and 48h can be adopted to test for memory-enhancing properties of the test compounds. Animals are administered the novel IRAP inhibitors intracerebroventricularly via a chronic indwelling cannula. These compounds are given 5 mins before the acquisition trial. The animals are then returned to their home cages for 24h or 48h and then tested again. The latency in entering the dark chamber will be a measure of the memory-enhancing property of the test compound.

### 4. Age-induced memory deficit model

Spatial learning impairment in aged rats is well documented and this deficit can be detected in the Barnes maze paradigm (Greferath *et al.,* 2000). The effect of IRAP inhibitors on aged-induced learning deficits can be tested in the Barnes maze paradigm.

For drug treatment, the animals are implanted with Alzet minipumps (secured subcutaneously in the neck region) which delivers the test compounds chronically into the cerebral ventricles.

Memory and learning can be tested in humans by any one of a number of well established neuropsychological tests such as California Verbal Learning Test, Wechsler Memory Scale-III, Hopkins Verbal Learning Test - Revised™, Rey Auditory Verbal Learning Test, and Rey-Osterrieth Complex Figure Design Test.

Other disorders and conditions include delayed labour and delayed and/or reduced lactation and disorders of glucose homeostasis, including hyperglycaemia and diabetes. The ability to regulate glucose uptake or homeostasis may have use in the treatment of a wide range of conditions. Insulin resistance leading to disregulation of glucose uptake or homeostasis can arise in diabetes, hypertension and AIDS and compounds contemplated herein may be useful in their treatment, in particular, the clinical sequalae associated with diabetes such as, diabetic retinopathy, obesity, kidney disease, circulatory problems and metabolic and heart disease.

Glucose uptake in insulin responsive 3T3 adipocyte cells can be measured and the method therefore is well known. Briefly, cells are incubated with the compounds in the absence and presence of increasing doses of insulin (0-100nmol). The incubation media also contains 50 µM 2-deoxy-D-[2,6-³H]glucose and the incubation is for 20-60 min at 37 C. At the end of the incubation period cells are washed and lysed, and the levels of 2-deoxy-D-[2,6-³H]glucose taken up by the cells measured utilising a liquid scintillation analyser.

Anti-diabetic activity can also be measured in animal models.

### (i) Type 2 diabetes

Animal models of type 2 diabetes including the Zucker diabetic fatty rats (ZDF) and ob/ob mice can be used for the testing of compounds for the ability to facilitate glucose homeostasis. Animals are fed or fasted and given an intraperitoneal (IP) injection of compound. Blood samples are collected by tail bleeding and glucose levels measured at various time intervals.

The effect of chronic treatment of IRAP inhibitors on glucose homeostasis in animal models of type 2 diabetes can be investigated by measuring the level of specific glycated proteins including haemoglobin and fructosamine in serum. Long term delivery of IRAP inhibitors is by osmotic mini-pump (doses 0-1mol) and blood samples are collected by tail bleed and glucose levels measured. At time of sacrifice levels of glycosylated haemoglobin and fructosamine are determined using standard procedures.

### (ii) Type 1 diabetes

Streptozotocin (STZ) treated animals are a model of type 1 diabetes. Blood samples collected by tail bleeding and glucose levels measured at various time intervals.

The effect of chronic treatment of IRAP inhibitors on glucose homeostasis in STZ rats is investigated by measuring the level of specific glycated proteins including haemoglobin and fructosamine in serum. Long term delivery of IRAP inhibitors is by osmotic mini-pump and blood samples are collected by tail bleed between 0-16 weeks and glucose levels measured. At time of sacrifice levels of glycosylated haemoglobin and fructosamine are determined using standard procedures.

Other disorders may involve other neurodegenerative disorders, such as motor neurone disease, progressive spinal muscular atrophy, disorders of the CNS, particularly those involving motor and sensory neurones or resulting form stroke or trauma, disorders of the cardiovascular system, including cardiac hypertrophy, congestive heart failure, hypertension and atherosclerosis, disorders of development and/or growth, disorders of the reproductive system or associated with pregnancy, including delayed labour and/or reduced lactation, and some cancers.

Subjects to be treated include mammalian subjects: humans, non-primates, livestock animals (including cows, horses, sheep, pigs and goats), companion animals (including dogs, cats, rabbits, guinea pigs), and captive wild animals. Laboratory animals such as rabbits, mice, rats, guinea pigs and hamsters are also contemplated as they may provide a convenient test system. Non-mammalian species such as birds, amphibians and fish may also be contemplated in certain embodiments of the invention.

The compounds of the invention are administered to the subject in an IRAP inhibiting, or treatment effective amount. An IRAP inhibiting amount is an amount which will at least partially interact with IRAP or disrupt IRAP activity. IRAP activity as used herein includes IRAP functional interaction with endogenous ligands, particularly where the functional interaction directly or indirectly promotes memory and/or learning loss, disrupts glucose homeostasis or delays labour or lactation in a subject. A treatment effective amount is intended to include an amount which, when administered according to the desired dosing regimen, at least partially attains the desired therapeutic or prophylactic effect of one or more of: alleviating the symptoms of, preventing or delaying the onset of, inhibiting the progression of, or halting or reversing, partially or altogether, the onset or progression of the particular disorder or condition being treated.

Suitable dosage amounts and dosing regimens can be determined by the attending physician and may depend on the particular condition being treated, the severity of the condition as well as the general age, health and weight of the subject.

The active ingredient may be administered in a single dose or a series of doses. While it is possible for the active ingredient to be administered alone, it is preferable to present it as a composition, preferably as a pharmaceutical composition, with one or more pharmaceutically acceptable adjuvants. Thus, the present invention also relates to the use of a compound of formula (I) or a pharmaceutically acceptable salt or prodrug thereof in the manufacture of a medicament for inhibiting IRAP activity in a subject.

The formulation of such compositions is well known to those skilled in the art, see for example, Remington's Pharmaceutical Sciences, 18th Edition, Mack Publishing, 1990. The composition may contain any suitable carriers, diluents or excipients. These include all conventional solvents, dispersion media, fillers, solid carriers, coatings, antifungal and antibacterial agents, dermal penetration agents, surfactants, isotonic and absorption agents and the like. It will be understood that the compositions of the invention may also include other supplementary physiologically active agents.

The carrier must be pharmaceutically acceptable in the sense of being compatible with the other ingredients of the composition and not injurious to the subject. Compositions include those suitable for oral, rectal, nasal, topical (including dermal, buccal and sublingual), vaginal or parental (including subcutaneous, intramuscular, intravenous and intradermal) administration. The compositions may conveniently be presented in unit dosage form and may be prepared by any methods well known in the art of pharmacy. Such methods include the step of bringing into association the active ingredient with the carrier which constitutes one or more accessory ingredients. In general, the compositions are prepared by uniformly and intimately bringing into association the active ingredient with liquid carriers or finely divided solid carriers or both, and then if necessary shaping the product.

Compositions of the present invention suitable for oral administration may be presented as discrete units such as capsules, sachets or tablets each containing a predetermined amount of the active ingredient; as a powder or granules; as a solution or a suspension in an aqueous or non-aqueous liquid; or as an oil-in-water liquid emulsion or a water-in-oil liquid emulsion.

A tablet may be made by compression or moulding, optionally with one or more accessory ingredients. Compressed tablets may be prepared by compressing in a suitable machine the active ingredient in a free-flowing form such as a powder or granules, optionally mixed with a binder (eg. inert diluent), preservative disintegrant (eg. sodium starch glycolate, cross-linked polyvinyl pyrrolidone, cross-linked sodium carboxymethyl cellulose) surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile. Tablets may optionally be provided with an enteric coating, to provide release in parts of the gut other than the stomach.

Compositions suitable for topical administration in the mouth include lozenges comprising the active ingredient in a flavoured base, usually sucrose and acacia or tragacanth gum; pastilles comprising the active ingredient in an inert basis such as gelatin and glycerin, or sucrose and acacia gum; and mouthwashes comprising the active ingredient in a suitable liquid carrier.

Compositions suitable for topical administration to the skin may comprise the compounds dissolved or suspended in any suitable carrier or base and may be in the form of lotions, gel, creams, pastes, ointments and the like. Suitable carriers include mineral oil, propylene glycol, polyoxyethylene, polyoxypropylene, emulsifying wax, sorbitan monostearate, polysorbate 60, cetyl esters wax, cetearyl alcohol, 2-octyldodecanol, benzyl alcohol and water. Transdermal patches may also be used to administer the compounds of the invention.

Compositions for rectal administration may be presented as a suppository with a suitable base comprising, for example, cocoa butter, glycerin, gelatin or polyethylene glycol.

Compositions suitable for vaginal administration may be presented as pessaries, tampons, creams, gels, pastes, foams or spray formulations containing in addition to the active ingredient such carriers as are known in the art to be appropriate.

Compositions suitable for parenteral administration (eg. subcutaneous, intramuscular, intravenous or intracerebroventricular) may include aqueous and non-aqueous isotonic sterile injection solutions which may contain anti-oxidants, buffers, bactericides and solutes which render the composition isotonic with the blood of the intended recipient; and aqueous and non-aqueous sterile suspensions which may include suspending agents and thickening agents. The compositions may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage compositions are those containing a daily dose or unit, daily sub-dose, as herein above described, or an appropriate fraction thereof, of the active ingredient.

It should be understood that in addition to the active ingredients particularly mentioned above, the compositions of this invention may include other agents conventional in the art having regard to the type of composition in question, for example, those suitable for oral administration may include such further agents as binders, sweeteners, thickeners, flavouring agents disintegrating agents, coating agents, preservatives, lubricants and/or time delay agents. Suitable sweeteners include sucrose, lactose, glucose, aspartame or saccharine. Suitable disintegrating agents include corn starch, methylcellulose, polyvinylpyrrolidone, xanthan gum, bentonite, alginic acid or agar. Suitable flavouring agents include peppermint oil, oil of wintergreen, cherry, orange or raspberry flavouring. Suitable coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propyl paraben or sodium bisulphite. Suitable lubricants include magnesium stearate, stearic acid, sodium oleate, sodium chloride or talc. Suitable time delay agents include glyceryl monostearate or glyceryl distearate.

The present invention also relates to prodrugs of the compounds described herein. Any compound that is a prodrug of a compound described herein is within the scope and spirit of the invention. The term "prodrug" is used in its broadest sense and encompasses those derivatives that are converted *in vivo,* either enzymatically or hydrolytically, to the compounds of the invention. Such derivatives would readily occur to those skilled in the art, and include, for example, compounds where a free thiol or hydroxy group is converted into an ester, such as an acetate, or where a free amino group is converted into an amide. Procedures for acylating the compounds of the invention, for example to prepare ester and amide prodrugs, are well known in the art and may include treatment of the compound with an appropriate carboxylic acid, anhydride or chloride in the presence of a suitable catalyst or base. Other conventional procedures for the selection and preparation of suitable prodrugs are known in the art and are described, for example, in WO 00/23419, Design of Prodrugs, Hans Bundgaard, Ed., Elsevier Science Publishers, 1985, and The Organic Chemistry of Drug Design and Drug Action, Chapter 8, pp352-401, Academic press, Inc., 1992, the contents of which are incorporated herein by reference.

Suitable pharmaceutically acceptable salts of compounds of formula (I) include, but are not limited to salts of pharmaceutically acceptable inorganic acids such as hydrochloric, sulphuric, phosphoric nitric, carbonic, boric, sulfamic, and hydrobromic acids, or salts of pharmaceutically acceptable organic acids such as acetic, propionic, butyric, tartaric, maleic, hydroxymaleic, fumaric, maleic, citric, lactic, mucic, gluconic, benzoic, succinic, oxalic, phenylacetic, methanesulphonic, toluenesulphonic, benezenesulphonic, salicyclic sulphanilic, aspartic, glutamic, edetic, stearic, palmitic, oleic, lauric, pantothenic, tannic, ascorbic and valeric acids. Base salts include, but are not limited to, those formed with pharmaceutically acceptable cations, such as sodium, potassium, lithium, calcium, magnesium, ammonium and alkylammonium. Basic nitrogen-containing groups may be quarternised with such agents as lower alkyl halide, such as methyl, ethyl, propyl, and butyl chlorides, bromides and iodides; dialkyl sulfates like dimethyl and diethyl sulfate; and others.

The compounds of the invention may be in crystalline form either as the free compounds or as solvates (for example, of water, ie. hydrates, or of common organic solvents such as alcohols) and it is intended that both forms are within the scope of the present invention. Methods of solvation are generally known within the art.

Tautomeric forms of compounds described herein, such as keto-enol tautomers, are also contemplated to be part of the invention where appropriate.

It will also be recognised that certain compounds for use in the invention may possess asymmetric centres and are therefore capable of existing in more than one stereoisomeric form. Where the compounds have at least 2 chiral centres, they may exist as diastereoisomers. The invention thus also relates to compounds in substantially pure isomeric form at one or more asymmetric centres, eg., enantiomers having greater than about 90% ee, such as about 95% or 97% ee or greater than 99% ee, as well as mixtures, including racemic mixtures, thereof. Such isomers may be prepared by asymmetric synthesis, for example using chiral intermediates, or mixtures may be resolved by conventional methods, eg., chromatography, or use of a resolving agent.

The compounds of the invention may also be used to treat non-human subjects and may therefore be presented as veterinary compositions. These may be prepared by any suitable means known in the art. Examples of such compositions include those adapted for:
(a) oral administration, external application (eg. drenches including aqueous and non-aqueous solutions or suspensions), tablets, boluses, powders, granules, pellets for admixture with feedstuffs, pastes for application to the tongue;
(b) parenteral administration, eg. subcutaneous, intramuscular, intravenous or intracerebroventricular injection as a sterile solution or suspension;
(c) topical application eg. creams, ointments, gels, lotions etc.

Those skilled in the art will appreciate that the invention described herein is susceptible to variations and modifications other than those specifically described. It is to be understood that the invention includes all such variations and modifications which fall within the spirit and scope. The invention also includes all of the steps, features, compositions and compounds referred to or indicated in this specification, individually or collectively, and any and all combinations of any two or more of said steps or features.

### EXAMPLES

### EXAMPLE 1: IN VITRO ASSAY FOR SCREENING FOR INHIBITORS OF IRAP

An in vitro enzymatic assay was used for screening for compounds which inhibit IRAP activity. The assay used an aminopeptidase substrate (leucine 4-methylcoumaryl-7-amide), a source of IRAP - containing material, and a candidate inhibitor.

### 1. Source of IRAP containing material

Potential sources of IRAP-containing material include cell lines or biological tissues expressing appreciable amounts of recombinant IRAP. Prokaryotic or eukaryotic cells expressing a transfected gene encoding IRAP represent a recombinant source.

### 2. Test Compounds

Test compounds were obtained from Specs, Delftechpark 30, 2628 XH Delft, The Netherlands. Compound 1 was also prepared according to methodology as described herein from resorcinol, pyridine 3-carboxaldehyde and ethyl cyanoacetate.

### 3. Monitoring the enzymatic activity of IRAP in the presence of a test substance

IRAP-containing material was incubated with a synthetic aminopeptidase substrate, such as Leu-MCA (leucine 4-methylcoumaryl-7-amide), and a test substance. Inhibition of the rate of degradation of the synthetic substrate was taken as a measure of the inhibitory effect of the test substance.

As a source of recombinant IRAP, HEK293 cells transfected with a eukaryotic expression vector pcDNA-3 containing the cDNA encoding the full length human IRAP were utilised. The assay system involved the measurement of the rate of hydrolysis of Leu-MCA by IRAP. The rate of hydrolysis of Leu-MCA, quantified by the release of a fluorigenic product, was monitored by recording an increase in fluorescence (excitation at 370nm, emission at 460nm) using a purpose-designed fluorimeter. Candidate test compounds were added to the assay system, and their effects on the hydrolysis of Leu-MCA were taken to be direct indicators of their ability to inhibit IRAP activity. The results are depicted in Table 1.

**TABLE 1**

| | | **IC₅₀ (M)** |
|---|---|---|
| 1 | 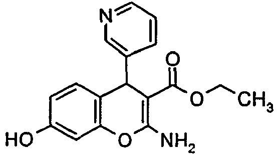 | 4.0 x 10⁻⁶ |
| 2 | 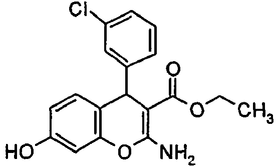 | 4.2 x 10⁻⁵ |
| 3 | 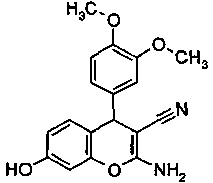 | 4.5 x 10⁻⁵ |
| 4 | 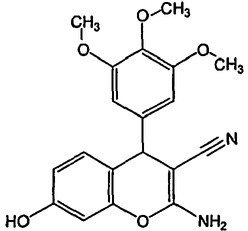 | 1 x 10⁻⁴ |
| 5 | 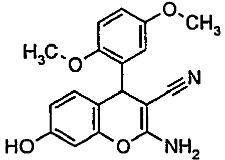 | 1 x 10⁻⁴ |
| 6 | 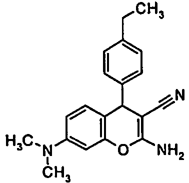 | 1 x 10⁻⁴ |
| 7 | 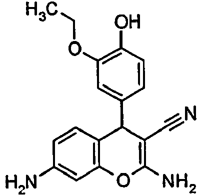 | 1 x 10⁻⁴ |
| 8 | 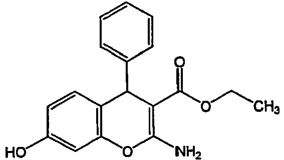 | 1 x 10⁻⁴ |
| 9 | 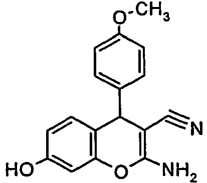 | 8 x 10⁻⁵ |
| 10 | 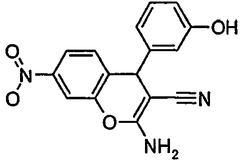 | 8 x 10⁻⁶ |

### EXAMPLE 2: IN VIVO ASSAY FOR SCREENING FOR COMPOUNDS WITH ANTI-AMNESIAC PROPERTIES IN ANIMAL MODELS OF AMNESIA

### 1 Scopolamine-induced memory deficit model

Scopolamine, a muscarinic receptor antagonist, has been used to induce amnesia in both rats and mice and demonstrated in a number of behavioural paradigms of memory and learning. This compound has been shown to impair short-term memory. The effect of IRAP inhibitor Compound 1 on scopolamine-induced amnesia was tested in the inhibitory avoidance paradigm.

The inhibitory avoidance box is made up of two chambers, a smaller light compartment and a larger darken compartment and the test comprises of two trials. The first training trial involves placing the rat in the light compartment facing the wall and as soon as it runs to the end of the dark compartment and turns around, an electric shock of 0.6 mA is administered for 2 s and the animal is left in the dark compartment for a total of 15 s before being returned to its home cage. After a 24h interval, the animal is then tested again and the latency in entering the dark chamber is a measure of the ability of the animal to remember the aversive stimulus. To test for the effect of the IRAP inhibitors, the rats were implanted with a chronic indwelling cannula into the lateral ventricles 1 week before the behavioural experiments. On the day of the testing, 15 min before the training trial, the rats were injected with either 70 nmol scopolamine hydrobromide dissolved in 2 ul of 0.9% saline or 0.9% saline before being placed in the inhibitory avoidance box for the training trial. Immediately after the training trial, the rats were injected with either 0.2 or 2 nmol of compound 1 (from Example 1) dissolved in 2 ul of 10% DMSO or vehicle control. The rats were then returned to their home cage and tested 24h later in the retrieval trial. Rats that were given either 0.2 or 2 nmol of compound 1 following scopolamine treatment exhibited increased latencies in entering the dark chamber compared to the group of rats treated with scopolamine followed by vehicle (Figure 1). The two doses of compound 1 both attenuated the memory deficit induced by scopolamine treatment, restoring the memory back to control levels (Figure 1).

### BIBLIOGRAPHY

Albiston et al., J Biol Chem 276: 48263-48266,2001.
Albiston et al., Behav Brain Res 154: 239-243, 2004.
Braszko, et al., Neuroscience 27: 777-783, 1988.
Bryant et al., Nat Rev Mol Cell Biol 3: 267-277, 2002.
Greferath et al., Neuroscience 100: 363-373, 2000.
Imamura et al., Am JPhysiol Regul Integr Comp Physiol 279: R1061-1067, 2000.
Keller et al., J Biol Chem 270: 23612-23618, 1995.
Lee et al., Neuroscience 124: 341-349, 2004.
Lew et al., Journal of Neurochemistry 86: 344-350, 2003.
Pederson et al., Regul Pept 74: 97-103, 1998.
Pederson et al., Regul Pept 102: 147-156, 2001.
Rogi et al., J Biol Chem 271: 56-61, 1996.
Waters et al., J Biol Chem 2 72: 23323-23327, 1997.
Wright et al., Brain Res Bull 32: 497-502, 1993.
Wright et al., Brain Res 717: 1-11, 1996.
Wright et al., JNeurosci 19: 3952-3961, 1999.
Zhang et al., J Pharmacol Exp Ther 289: 1075-1083, 1999.

## Claims

1. A compound for use in treating memory loss or impairment, or for use in enhancing memory and/or learning in a subject, wherein the compound has the Formula: 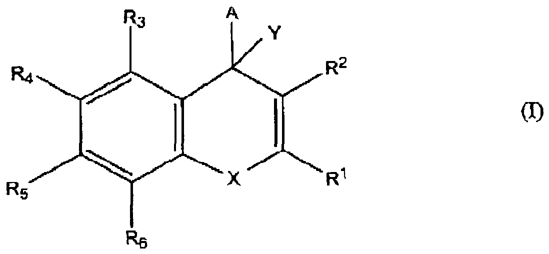 wherein
A is aryl, heteroaryl carbocyclyl or heterocyclyl, each of which may be optionally substituted;
X is O;
R¹ is NR⁷R⁸, NHCOR₈, N(COR₈)₂, N(COR₇)(COR₈), N=CHOR₈ or N=CHR₈ wherein R⁷ and R⁸ are independently selected from H, optionally substituted alkyl, optionally substituted aryl, or R⁷ and R⁸, together with the nitrogen atom to which they are attached form a 3-8-membered ring which may be optionally substituted;
R² is CN, CO₂R⁹, C(O)O(O)R⁹, C(O)R⁹ or C(O)NR⁹R¹⁰ wherein R⁹ and R¹⁰ are independently selected from alkyl, alkenyl, alkynyl, aryl, heteroaryl, carbocyclyl, heterocyclyl, each of which may be optionally substituted, and hydrogen, or R⁹ and R¹⁰, together with the nitrogen atom to which they are attached, form a 3-8-membered ring which may be optionally substituted;
R³-R⁶ are independently selected from hydrogen, halo, nitro, cyano alkyl, alkenyl, alkynyl, aryl, heteroaryl, heterocyclyl, carbocyclyl, hydroxy, alkoxy, alkenyloxy, alkynyloxy, alkynyloxy, aryloxy, heteroaryloxy, haterocyclyloxy, amino, acyl, acyloxy, carboxy, carboxyester, methylenedioxy, amido, thio, alkylthio, alkenylthio, alkynylthio, arylthio, heteroarylthio, heterocyclylthio, carbocyclylthio, acylthio and azido, each of which may be optionally substituted where appropriate, or any two adjacent R³-R⁶, together with the atoms to which they are attached, form a 3-8-membered ring which may be optionally substituted; and
Y is hydrogen or C₁₋₁₀alkyl,
or a pharmaceutically acceptable salt thereof.

2. The compound for use according to claim 1 wherein A is aryl or heteroaryl.

3. The compound for use according to claim 1 wherein A is 5-6-membered.

4. The compound for use according to claim 3 wherein A is optionally substituted 5-6-membered aryl or heteroaryl.

5. The compound for use according to claim 4 wherein A is optionally substituted phenyl or pyridyl.

6. The compound for use according to any one of claims 1 to 5 wherein Y is hydrogen.

7. The compound for use according to any one of claims 1 to 6 wherein R¹ is NR⁷R⁸.

8. The compound for use according to any one of claims 1 to 7 wherein R¹ is NH₂.

9. The compound for use according to any one of claims 1 to 8 wherein R² is CN, CO₂R⁹ or C(O)NR⁹R¹⁰ or C(O)R⁹.

10. The compound for use according to any one of claims 1 to 9 wherein R³-R⁶ are selected from H, OH, CN, halo, C₁₋₁₀alkyl, C₂₋₁₀alkenyl, C₁₋₁₀alkoxy, phenyl, halophenyl, hydroxyphenyl, aminophenyl, alkylphenyl, NH₂, NH C₁₋₁₀alkyl, NC₁₋₁₀alkyl C₁₋₁₀alkyl, NO₂, ⁺NO₂H, haloalkyl, acyl, acyloxy carboxy ester, CO₂H, amido, thio, alkylthio, and 2 of adjacent R³-R⁶ form methylenedioxy.

11. The compound for use according to claim 10 wherein R₃, R₄ and R₆ are all hydrogen.

12. The compound for use according to claim 10 wherein R⁵ is selected from OH, CN, halo, C₁₋₁₀alkyl, NO₂, ⁺NO₂H, NH₂, NHC₁₋₁₀alkyl, NC₁₋₁₀alkyl C₁₋₁₀alkyl.

## Patentansprüche

1. Verbindung zur Verwendung beim Behandeln von Verlust oder Beeinträchtigung des Gedächtnisses oder zur Verwendung beim Verbessern des Gedächtnisses und/oder des Lernens bei einem Patienten, wobei die Verbindung die Formel 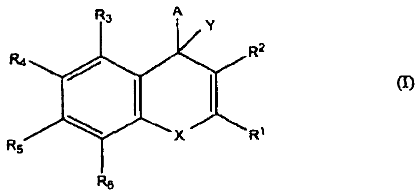 hat, wobei
A Aryl, Heteroaryl, Carbocyclyl oder Heterocyclyl ist, von welchen jedes wahlweise substituiert sein kann;
X O ist;
R¹ NR⁷R⁸, NHCOR₈, N(COR₈)₂, N(COR₇)(COR₈), N=CHOR₈ oder N=CHR₈ ist, wobei R⁷ und R⁸ unabhängig aus H, wahlweise substituiertem Alkyl, wahlweise substituiertem Aryl ausgewählt sind oder R⁷ und R⁸, zusammen mit dem Stickstoffatom, an welches sie gebunden sind, einen 3―8-gliedrigen Ring bilden, welcher wahlweise substituiert sein kann;
R² CN, CO₂R⁹, C(O)O(O)R⁹, C(O)R⁹ oder C(O)NR⁹R¹⁰ ist, wobei R⁹ und R¹⁰ unabhängig aus Alkyl, Alkenyl, Alkinyl, Aryl, Heteroaryl, Carbocyclyl, Heterocyclyl, von welchen jedes wahlweise substituiert sein kann, und Wasserstoff ausgewählt sind oder R⁹ und R¹⁰, zusammen mit dem Stickstoffatom an welches sie gebunden sind, einen 3―8-gliedrigen Ring bilden, welcher wahlweise substituiert sein kann;
R³―R⁶ unabhängig aus Wasserstoff, Halogen, Nitro, Cyano, Alkyl, Alkenyl, Alkinyl, Aryl, Heteroaryl, Heterocyclyl, Carbocyclyl, Hydroxy, Alkoxy, Alkenyloxy, Alkinyloxy, Alkinyloxy, Aryloxy, Heteroaryloxy, Heterocyclyloxy, Amino, Acyl, Acyloxy, Carboxy, Carboxyester, Methylendioxy, Amido, Thio, Alkylthio, Alkenylthio, Alkinylthio, Arylthio, Heteroarylthio, Heterocyclylthio, Carbocyclylthio, Acylthio und Azido ausgewählt sind, von welchen jedes wahlweise, wo es passend ist, substituiert sein kann, oder zwei benachbarte R³―R⁶, zusammen mit den Atomen, an welche sie gebunden sind, einen 3―8-gliedrigen Ring bilden, welcher wahlweise substituiert sein kann; und
Y Wasserstoff oder C₁₋₁₀Alkyl ist,
oder ein pharmazeutisch verträgliches Salz davon.

2. Verbindung zur Verwendung gemäß Anspruch 1, wobei A Aryl oder Heteroaryl ist.

3. Verbindung zur Verwendung gemäß Anspruch 1, wobei A 5―6-gliedrig ist.

4. Verbindung zur Verwendung gemäß Anspruch 3, wobei A wahlweise substituiertes 5―6-gliedriges Aryl oder Heteroaryl ist.

5. Verbindung zur Verwendung gemäß Anspruch 4, wobei A wahlweise substituiertes Phenyl oder Pyridyl ist.

6. Verbindung zur Verwendung gemäß einem der Ansprüche 1 bis 5, wobei Y Wasserstoff ist.

7. Verbindung zur Verwendung gemäß einem der Ansprüche 1 bis 6, wobei R¹ NR⁷R⁸ ist.

8. Verbindung zur Verwendung gemäß einem der Ansprüche 1 bis 7, wobei R¹ NH₂ ist.

9. Verbindung zur Verwendung gemäß einem der Ansprüche 1 bis 8, wobei R² CN, CO₂R⁹ oder C(O)NR⁹R¹⁰ oder C(O)R⁹ ist.

10. Verbindung zur Verwendung gemäß einem der Ansprüche 1 bis 9, wobei R³―R⁶ aus H, OH, CN, Halogen, C₁₋₁₀Alkyl, C₂―₁₀Alkenyl, C₁₋₁₀Alkoxy, Phenyl, Halogenphenyl, Hydroxyphenyl, Aminophenyl, Alkylphenyl, NH₂, NHC₁₋₁₀Alkyl, NC₁₋₁₀AlkylC₁₋₁₀alkyl, NO₂, ⁺NO₂H, Halogenalkyl, Acyl, Acyloxy, Carboxyester, CO₂H, Amido, Thio, Alkylthio ausgewählt sind und 2 von benachbarten R³-R⁶ Methylendioxy bilden.

11. Verbindung zur Verwendung gemäß Anspruch 10, wobei R₃, R₄ und R₆ alle Wasserstoff sind.

12. Verbindung zur Verwendung gemäß Anspruch 10, wobei R⁵ aus OH, CN, Halogen, C₁₋₁₀Alkyl, NO₂, ⁺NO₂H, NH₂, NHC₁₋₁₀Alkyl, NC₁₋₁₀AlkylC₁₋₁₀alkyl ausgewählt ist.

## Revendications

1. Composé pour une utilisation dans le traitement d'une perte ou d'une déficience de la mémoire ou pour une utilisation dans l'amélioration de la mémoire et/ou de l'apprentissage chez un sujet, où le composé présente la Formule: 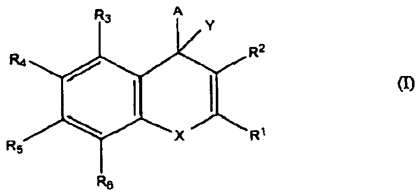 dans laquelle:
A est un aryle, un hétéroaryle, un carbocyclyle ou un hétérocyclyle, chacun pouvant être optionnellement substitué;
X est O;
R¹ est NR⁷R⁸, NHCOR₈, N(COR₈)₂, N(COR₇)(COR₈), N=CHOR₈ ou N=CHR₈, où R⁷ et R⁸ sont indépendamment choisis parmi H, un alkyle optionnellement substitué, un aryle optionnellement substitué, ou R⁷ et R⁸, accompagnés de l'atome d'azote auquel ils sont attachés, forment un cycle de 3-8 membres qui peut être optionnellement substitué;
R² est CN, CO₂R⁹, C(O)O(O)R⁹, C(O)R⁹ ou C(O)NR⁹R¹⁰, où R⁹ et R¹⁰ sont indépendamment choisis parmi un alkyle, un alcényle, un alcynyle, un aryle, un hétéroaryle, un carbocyclyle, un hétérocyclyle, chacun pouvant être optionnellement substitué, et un hydrogène, ou R⁹ et R¹⁰, accompagnés de l'atome d'azote auquel ils sont attachés, forment un cycle de 3-8 membres qui peut être optionnellement substitué;
R³-R⁶ sont indépendamment choisis parmi un hydrogène, un halo, un nitro, un cyano, un alkyle, un alcényle, un alcynyle, un aryle, un hétéroaryle, un hétérocyclyle, un carbocyclyle, un hydroxy, un alcoxy, un alcényloxy, un alcynyloxy, un alcynyloxy, un aryloxy, un hétéroaryloxy, un hétérocylyloxy, un amino, un acyle, un acyloxy, un carboxy, un carboxyester, un méthylènedioxy, un amido, un thio, un alkylthio, un alcénylthio, un alcynylthio, un arylthio, un hétéroarylthio, un hétérocyclylthio, un carbocyclylthio, un acylthio et un azido, chacun pouvant être optionnellement substitué lorsque c'est approprié, ou n'importe quels deux R³-R⁶ adjacents, accompagnés des atomes auxquels ils sont attachés, forment un cycle de 3-8 membres qui peut être optionnellement substitué; et
Y est un hydrogène ou un alkyle C₁₋₁₀,
ou un sel pharmaceutiquement acceptable de celui-ci.

2. Composé pour une utilisation selon la revendication 1, dans lequel A est un aryle ou un hétéroaryle.

3. Composé pour une utilisation selon la revendication 1, dans lequel A est de 5-6 membres.

4. Composé pour une utilisation selon la revendication 3, dans lequel A est un aryle ou un hétéroaryle de 5-6 membres optionnellement substitué.

5. Composé pour une utilisation selon la revendication 4, dans lequel A est un phényle ou un pyridyle optionnellement substitué.

6. Composé pour une utilisation selon l'une quelconque des revendications 1 à 5, dans lequel Y est un hydrogène.

7. Composé pour une utilisation selon l'une quelconque des revendications 1 à 6, dans lequel R¹ est NR⁷R⁸.

8. Composé pour une utilisation selon l'une quelconque des revendications 1 à 7, dans lequel R¹ est NH₂.

9. Composé pour une utilisation selon l'une quelconque des revendications 1 à 8, dans lequel R² est CN, CO₂R⁹ ou C(O)NR⁹R¹⁰ ou C(O)R⁹.

10. Composé pour une utilisation selon l'une quelconque des revendications 1 à 9, dans lequel R³-R⁶ sont choisis parmi H, OH, CN, un halo, un alkyle C₁₋₁₀, un alcényle C₂₋₁₀, un alcoxy C₁₋₁₀, un phényle, un halophényle, un hydroxyphényle, un aminophényle, un alkylphényle, NH₂, un NH-alkyle C₁₋₁₀, un N-alkyle C₁₋₁₀-alkyle C₁₋₁₀, NO₂, ⁺NO₂H, un haloalkyle, un acyle, un acyloxy, un carboxyester, CO₂H, un amido, un thio, un alkylthio, et 2 de R³-R⁶ adjacents forment un méthylènedioxy.

11. Composé pour une utilisation selon la revendication 10, dans lequel R₃, R₄ et R₆ sont tous des hydrogènes.

12. Composé pour une utilisation selon la revendication 10, dans lequel R⁵ est choisi parmi OH, CN, un halo, un alkyle C₁₋₁₀, NO₂, ⁺NO₂H, NH₂, un NH-alkyle C₁₋₁₀, un N-alkyle C₁₋₁₀-alkyle C₁₋₁₀.
